# EUROPEAN PATENT APPLICATION

(11) **EP 0 782 854 A2**
(43) Date of publication of application: **09.07.1997**
(21) Application number: 96116343.3
(22) Date of filing: 11.10.1996
(51) Int. Cl.: A61K 31/00

(54) **Use of isopropyl-2-(1,3-dithietan-2-ylidene)-2-(N-(4-methylthiazol-2-yl)carbamoyl)acetate as a cancer chemopreventive agent**

(30) Priority: 14.10.1995 KR 9535537
(71) Applicant: Yuhan Corporation, Seoul 150-020 (KR)
(72) Inventor: Lee, Jong-Wook, Kwacheon-si, Kyonggi-do 427-040 (KR); Yoo, Joong-Keun, Kunpo-si, Kyonggi-do 435-042 (KR); Lee, Sang-Ho, Dongan-gu, Anyang-si, Kyonggi-do 430-060 (KR)
(74) Representative: von Samson-Himmelstjerna, Friedrich R., Dipl.-Phys.

(57) **Abstract**

A use of isopropyl 2-(1,3-dithietan-2-ylidene)-2-[N-(4-methylthiazol-2-yl)carbamoyl]acetate and its pharmaceutically acceptable salts in the manufacture of medicine for the prevention of cancer.

## Description

### Field of the Invention

The present invention relates to a novel use of isopropyl 2-(1,3-dithietan-2-ylidene)-2-[N-(4-methylthiazol-2-yl)carbamoyl]acetate and its pharmaceutically acceptable salts, as a cancer chemopreventive agent.

### Description of the prior Art

While most anti-cancer agents are intended for use in chemotherapy of cancer patients, there have been recent attempts to develop cancer chemopreventive agents. A cancer chemopreventive agent, as used herein, refers to a compound, or a pharmaceutical composition containing same as the active ingredient, which is effective in preventing cancer.

Many compounds, including some compounds previously developed for other therapeutic purpose, are now known to have cancer chemopreventive activities. For example, olipraz, 5-(2-pyrazinyl)-4-methyl-1,2-dithiol-3-one, which is initially used as an antischistosomal drug, has been found to possess inhibitory effect on chemically induced carcinogenesis, and it is now being developed as a cancer chemopreventive agent by American National Cancer Research Center [see V. R. Chinthalapally, K. Tokomo, G. Kelloff and B.S. Reddy, Carcinogenesis, 12, pp. 1051-1055(1991)].

The present inventors have previously developed thioketene derivatives and non-toxic salts thereof which possess excellent therapeutic and prophylactic activities for hepatic diseases (see United States Patent No. 5,103,013). Among those compounds, isopropyl 2-(1,3-dithietan-2-ylidene)-2-[N-(4-methylthiazol-2-yl)carbamoyl] acetate and its pharmaceutically acceptable salts have been found, quite unexpectedly, to possess excellent cancer chemopreventive activities.

### Summary of the Invention

Accordingly, it is a primary object of the present invention to provide a novel use of isopropyl 2-(1,3-dithietan-2-ylidene)-2-[N-(4-methylthiazol-2-yl)carbamoyl] acetate and its pharmaceutically acceptable salts as a cancer chemopreventive agent.

In accordance with the present invention, there is provided a novel use of isopropyl 2-(1,3-dithietan-2-ylidene)-2-[N-(4-methylthiazol-2-yl)carbamoyl]acetate and its pharmaceutically acceptable salts in the manufacture of medicine for the prevention of cancer.

### Brief Description of the Drawing

The above and other objects and features of the present invention will become apparent from the following description thereof, when taken in conjunction with the accompanying drawing wherein:

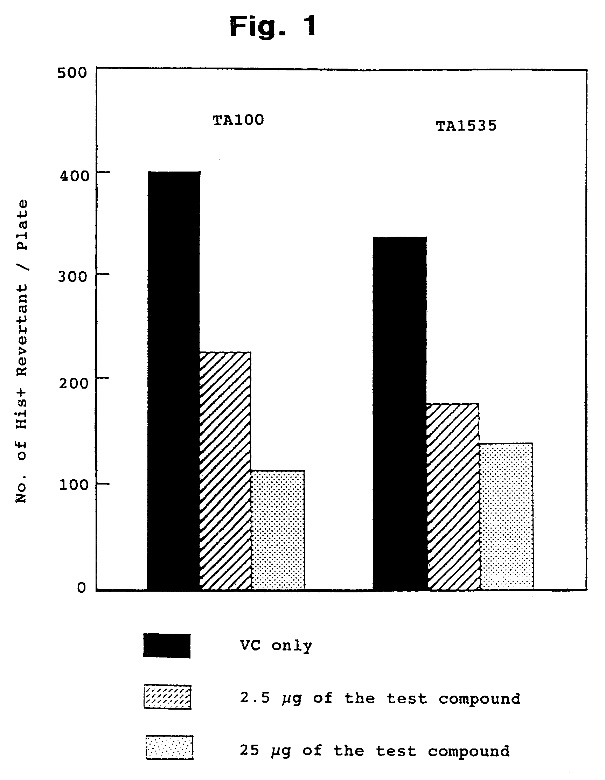

Fig. 1 shows the effect of isopropyl 2-(1,3-dithietan-2-ylidene)-2-[N-(4-methylthiazol-2-yl)carbamoyl]acetate on mutagenesis of TA98 and TA100 induced by vinyl carbamate.

### Detailed Description of the Invention

Isopropyl 2-(1,3-dithietan-2-ylidene)-2-[N-(4-methylthiazol-2-yl)carbamoyl]acetate of formula(I) and its pharmaceutically acceptable salts can be tested for their cancer chemopreventive activities by employing a mutagenesis test, covalent DNA binding test and mouse skin tumor test.

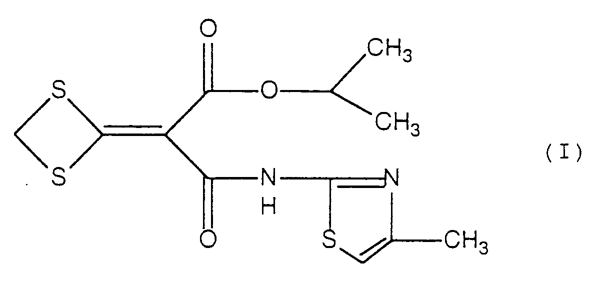

Benzo[a]pyrene(a prototypic environmental carcinogen, "B[a]P") is activated by monooxygenases, particularly by those in cytochromeP1A and cytochromeP3A which are subfamilies of cytochromeP450(CYP), to form an electrophilic epoxide, i.e., benzo[a]pyrene-7,8-dihydrodiol-9,10-epoxide(BPDE) which possesses mutagenic and tumorigenic activities[see J. M. Sayer, Whalen and D.M. Jerina, Drug Metab. Rev., 20, pp. 155-182(1989)]. Further, Vinyl carbamate(VC) is activated by cytochrome P2E1 to an ultimate electrophilic and carcinogenic epoxide metabolite(VC epoxide)[see F. P. Guengerich, D.H. Kim and M. Iwasaki, Chem. Res. Toxicol., 4, pp. 168-179(1991)].

Isopropyl 2-(1,3-dithietan-2-ylidene)2-[N-(4-methylthiazol-2-yl)carbamoyl]acetate and its pharmaceutically acceptable salts can effectively inhibit: mutagenesis induced by benzo[a]pyrene, benzo[a]pyrene-7,8-dihydrodiol-9,10-epoxide, vinyl carbamate and vinyl carbamate epoxide; covalent binding of benzo[a]pyrene to DNA; and mouse skin tumor, as is described in the Test Examples. Accordingly, isopropyl 2-(1,3-dithietan-2-ylidene)-2-[N-(4-methylthiazol-2-yl)carbamoyl] acetate and its pharmaceutically acceptable salts can be used for preventing cancer.

Isopropyl 2-(1,3-dithietan-2-ylidene)2-[N-(4-methylthiazol-2-yl)carbamoyl]acetate and its pharmaceutically acceptable salts can be readily prepared by the method described in United States Patent No. 5,103,013.

The present invention also provides a pharmaceutical composition for preventing cancer which comprises an effective amount of isopropyl 2-(1,3-dithietan-2-ylidene)-2-[N-(4-methylthiazol-2-yl)carbamoyl]acetate or its pharmaceutically acceptable salt and pharmaceutically acceptable carriers.

The pharmaceutical composition in accordance with the present invention may be prepared in various dosage forms using pharmaceutically acceptable carriers. The pharmaceutically acceptable carriers which may be used in the present invention include, but not limited to, suitable diluents, solvents, fillers, binders, dispersants, disintegrants, surfactants, lubricants, excipients and wetting agents. The present composition may further include solubilizers, buffers, preservatives, coloring agents, perfumes, sweeteners and the like, if necessary.

The pharmaceutical formulation of the present invention may be prepared in accordance with the desired mode of treatment. For example, oral dosage forms such as tablets, capsules and granules, may be prepared by employing any conventional method by using diluents(e.g.: sucrose, lactose, glucose, starch, mannitol), binders(e.g.: syrup, arabia gum, sorbitol, tragacanth gum, hydroxypropyl-cellulose, polyvinylpyrrolidone, starch paste); disintegrants(e.g.: starch, carboxymethylcellulose or its calcium salt, microcrystalline cellulose, crospovidone, starch-sodium glycolate); lubricants(e.g.: talc, magnesium stearate, calcium stearate, silica); wetting agents(e.g.: sodium laurylsulfate, glycerol) and the like.

The present invention further provides a method for preventing cancer in a subject by administering an effective amount of isopropyl 2-(1,3-dithietan-2-ylidene)-2-[N-(4-methylthiazol-2-yl)carbamoyl]acetate or its pharmaceutically acceptable salt to the subject.

The dosage may be varied depending on the method of administration and the formulation type as well as the age, body weight, sensitivity and condition of the patient. In case of an oral administration, an effective daily dosage may range, e.g., from 20mg to 1.0g. A single dosage unit which contains isopropyl 2-(1,3-dithietan-2-ylidene)-2-[N-(4-methylthiazol-2-yl)carbamoyl]acetate or its pharmaceutically acceptable salt in an amount ranging from 20mg to 200mg may be conveniently used to meet the required daily dosage. The dose and dosage units may be used beyond the above scope.

The following Test Examples are provided for the purpose of illustrating certain aspects of the present invention; and are not to be construed as limiting the scope of the present invention in any way.

### Test Example 1: Mutagenesis Test

Cancer chemopreventive activity of isopropyl 2-(1,3-dithietan-2-ylidene)-2-[N-(4-methylthiazol-2-yl)carbamoyl] acetate on mutagenesis induced by B[a]P, BPDE, VC and VC epoxide was evaluated as follows.

Male Sprague-Dawley rats (40 to 45 day old) were fed with a standard laboratory pellet diet and water ad libitum under standard conditions(12h light-12h dark cycle, temp.: 21±2°C). They were treated intraperitoneally with Aroclor 1254 (500mg/kg in corn oil) for 5 consecutive days prior to sacrifice. Rats were killed by CO₂ asphyxiation, and their livers were immediately removed and perfused in situ with ice-cooled isotonic KCl solution. The liver tissue was weighed, minced, and homogenized in 3-fold volumes of ice-cooled 0.25M sucrose solution by employing a motorized Potter-Elvehjem glass-Teflon homogenizer. The homogenates were centrifuged at 9,000 X g for 30 minutes and the supernatant(S9 fraction) was used as the source of enzymes.

### 1-1. Mutagenesis induced by B[a]P

The Ames His+ reversion assay was performed in accordance with the liquid preincubation method using Salmonella typhimurium TA98 and TA100 as test strains[see D.M. Maton and B. N. Ames, Mutation Res., 113, pp. 173-221(1983)].

Each of TA98 and TA100(1 to 2 x 10⁹/plate) and 2.5µg of B[a]P dissolved in DMSO were added to the Aroclor 1254-induced rat hepatic postmitochondrial supernatant containing NADPH-generating system obtained above(50µg containing about 2mg protein). Various amounts of isopropyl 2-(1,3-dithietan-2-ylidene)-2-[N-(4-methylthiazol-2-yl)carbamoyl]acetate were added thereto, and preincubated at 37°C for 20 minutes.

The mixture was then diluted with soft agar, poured onto hard agar plates, and incubated for another 48 hours to allow the development of His+ revertant colonies. The data were corrected for the numbers of spontaneous revertant. The results are shown in Table I.

**Table I**

| Compound* (µg) | No. of His+ revertant/plate | |
|---|---|---|
| | TA98 | TA100 |
| 0 | 116±3.6 | 248±26.0 |
| 1 | 84±16.1 | 205±23.1 |
| 5 | 48±3.2 | 140±4.6 |
| 25 | 23±2.5 | 93±8.7 |

| | | |
|---|---|---|
| *: Compound: isopropyl2-(1,3-dithietan-2-ylidene)-2-[N-(4-methylthiazol-2-yl)carbamoyl]acetate. | | |

### 1-2. Mutagenesis induced by BPDE

The Ames His+ reversion assay was performed in accordance with the liquid preincubation method using Salmonella typhimurium TA100 as a test strain[see D.M. Maton and B. N. Ames, Mutation Res., 113, pp. 173-221(1983)].

25nmol of BPDE was incubated with S. typhimurium TA100 in the absence or presence of a given amount of isopropyl 2-(1,3-dithietan-2-ylidene)-2-[N-(4-methylthiazol-2- yl)carbamoyl]acetate at 37 °C for 20 minutes.

Then, the mixture was diluted with soft agar, poured onto hard agar plates, and incubated for another 48 hours to allow the development of His+ revertant colonies. The results are shown in Table II. The data are expressed as average of values from duplicate analysis.

**Table II**

| Compound* (µg) | No. of His+ revertant/plate | % inhibition |
|---|---|---|
| 0 | 706 | - |
| 2.5 | 301 | 58 |
| 10 | 217 | 69 |
| 25 | 180 | 74 |

| | | |
|---|---|---|
| *: Compound: isopropyl2-(1,3-dithietan-2-ylidene)-2-[N-(4-methylthiazol-2-yl)carbamoyl]acetate. | | |

### 1-3. Mutagenesis induced by VC

The procedure of Test Example 1-1 was repeated except that Salmonella typhimurium TA100 and TA1535 as test strains, vinyl carbamate(10µmol) as a mutagen and 2.5µg and 25µg of isopropyl-2-(1,3-dithietan-2-ylidene)-2-[N-(4-methylthiazol-2-yl)carbamoyl]acetate were used.

The effect of the present compound on mutagenesis induced by VC are shown in Figure 1.

### 1-4. Mutagenesis induced by VC epoxide

The procedure of Test Example 1-2 was repeated except that Salmonella typhimurium TA100 was used as a test strain and vinyl carbamate epoxide(25 and 50nmol) was used as mutagens.

The effect of the present compound on mutagenesis induced by VC epoxide are shown in Table III.

**Table III**

| Compound* (µg) | No. of His+ revertant/plate | |
|---|---|---|
| | 25 nmol | 50 nmol |
| 0 | 283 | 598 |
| 2.5 | 247 | 541 |
| 10 | 239 | 509 |
| 25 | 201 | 471 |

| | | |
|---|---|---|
| *: Compound: isopropyl2-(1,3-dithietan-2-ylidene)-2-[N-(4-methylthiazol-2-yl)carbamoyl]acetate. | | |

As shown in Tables I, II, III and Figure 1, isopropyl 2-(1,3-dithietan-2-ylidene)-2-[N-(4-methylthiazol-2- yl)carbamoyl]acetate can dose-dependently inhibit the mutagenesis of bacteria induced by prototypic mutagens, i.e., B[a]p, BPDE, VC and VC epoxide.

### Test Example 2: Covalent binding test

2ml of the mixture of 50mM potassium phosphate buffer (pH 7.4) containing 0.25µg of [³H]-B[a]P(specific activity: 12.35mCi/mmol), 2mg of calf thymus DNA, 25mg of EDTA, NADPH(0.65mM), and 4 mg of hepatic postmitochondrial supernatant was incubated with isopropyl 2-(1,3-dithietan-2-ylidene)-2-[N-(4-methylthiazol-2yl)carbamoyl]acetate of various concentrations at 37°C for 30 minutes.

DNA was isolated by ethanol precipitation after the removal of proteins. DNA pellets thus obtained were successively rinsed with 75% ethanol containing 1% NaCl, 75% ethanol, absolute ethanol, acetone and ethyl ether, and then dried.

Dried DNA samples were dissolved in 15 mM NaCl-1.5mM sodium citrate buffer(pH 7.0). The DNA concentration was determined by diphenylamine method using calf thymus DNA as a standard[see K. Burton and G.B. Peterson, Biochem. J., 75, pp.17-27, 1960].

Aliqouts of DNA solutions were dissolved in Solvable(Du Pont/NEN Research Products, Boston, MA). After neutralization with glacial acetic acid, the levels of radio-labeled hydrocarbons covalently bound to DNA were determined by employing a liquid scintillation counter with the addition of Liquescent(National Diagnostics, Manville, NJ) as a cocktail. The results are shown in Table IV.

**Table IV**

| Compound* (µM) | Covalent DNA binding (pmol) |
|---|---|
| 0 | 60±7.1 |
| 5 | 22±2.0 |
| 50 | 16±3.2 |

| | |
|---|---|
| *: Compound: isopropyl 2-(1,3-dithietan-2-ylidene)-2-[N-(4-methylthiazol-2-yl)carbamoyl]acetate. | |

As shown in Tables IV, isopropyl 2-(1,3-dithietan-2-ylidene)-2-[N-(4-methylthiazol-2-yl)carbamoyl]acetate can dose-dependently inhibit the covalent binding of B[a]P to calf thymus DNA.

### Test Example 3: Mouse skin tumor test

Inhibitory effect of isopropyl 2-(1,3-dithietan-2-ylidene)-2-[N-(4-methylthiazol-2-yl)carbamoyl]acetate on mouse skin tumor was also investigated. Diallyl sulfide, which is known to possess cancer chemopreventive activity, was used as a comparative.

### 3-1. Carcinogenesis induced by VC

The dorsal region of female ICR mice (6- to 7-week old) was shaved. Groups of 30 mice were administered with 2.5µmol isopropyl 2-(1,3-dithietan-2-ylidene)-2-[N-(4-methylthiazol-2-yl)carbamoyl]acetate in 0.2% sodium carboxymethyl cellulose or 8µmol of diallyl sulfide in 0.1M sterilized potassium phosphate buffer. After 10 minutes, the mice were administered topically with VC(5.8 µmol) in 0.2ml acetone containing 15% DMSO. Control group was administered with VC alone.

One week after the initiation, the mice were administered topically with 15nmol TPA(12-O-tetradecanoyl phorbol-13-acetate)in 0.1 ml acetone twice a week to promote carcinogenic process.

Tumors of at least 1 mm in diameter were counted and recorded every other week. The results are shown in Table V. Numbers in parentheses indicate the % of tumor bearing mice.

**Table V**

| Pretreatment | VC | Average No. of papillomas/mouse (% of tumor bearing mice) | | | |
|---|---|---|---|---|---|
| | | time(week) | | | |
| | | 14 | 16 | 18 | 20 |
| - | - | 0 | 0 | 0 | 0 |
| - | + | 0.9±1.1 (53) | 1.4±1.7 (63) | 1.8±1.9 (67) | 2.4±2.1 (70) |
| Compound* | + | 0.2±0.5 (20) | 0.4±0.7 (30) | 0.6±0.9 (40) | 0.8±1.0 (50) |
| Diallyl sulfide | + | 0.2±0.5 (17) | 0.5±0.9 (30) | 0.7±1.1 (37) | 0.9±1.1 (50) |

| | | | | | |
|---|---|---|---|---|---|
| *: Compound: isopropyl 2-(1,3-dithietan-2-ylidene)-2-[N-(4-methylthiazol-2-yl)carbamoyl]acetate | | | | | |

### 3-2. Carcinogenesis induced by B[a]P or VC

trioctanoin, or isopropyl 2-(1,3-dithietan-2-ylidene)-2-[N-(4-methylthiazol-2-yl)carbamoyl]acetate(0.15mg/10µl/g body wt.) or diallyl sulfide(0.2mg/10µl/g body wt.) suspended in trioctanoin were administered to female CD-1 mice(6 weeks old) by gastric gavage method 24 hours and 1 hour prior to the intraperitoneal injection of B[a]P(0.3µmol) or VC(11.5µmol) in 0.2ml acetone or the same volume of acetone alone.

One week after the initiation, animals were administered topically with 15nmol TPA(12-O-tetradecanoyl phorbol-13-acetate) in 0.1ml acetone twice a week to promote carcinogenesis.

Tumors of at least 1 mm in diameter were counted and recorded at 16 weeks after initiation. The results are shown in Table VI.

**Table VI**

| Pretreatment | Initiator | No. of mice at 16 week | % mice with papillomas | Ave. No. of tumor/mouse |
|---|---|---|---|---|
| Trioctanoin | Acetone | 20 | 0 | 0 |
| Compound* | Acetone | 25 | 0 | 0 |
| Diallyl sulfide | Acetone | 25 | 0 | 0 |
| Trioctanoin | B[a]P | 25 | 92 | 6.7±5.1 |
| Compound* | B[a]P | 25 | 72 | 2.8±3.4 |
| Diallyl sulfide | B[a]P | 25 | 64 | 4.0±5.6 |
| Trioctanoin | VC | 25 | 58 | 1.0±1.6 |
| Compound* | VC | 25 | 36 | 0.5±0.8 |
| Diallyl sulfide | VC | 25 | 28 | 0.4±0.9 |

| | | | | |
|---|---|---|---|---|
| *: Compound: isopropyl 2-(1,3-dithietan-2-ylidene)-2-[N-(4-methylthiazol-2-yl)carbamoyl]acetate | | | | |

As shown in Tables V and VI, pretreatment of isopropyl 2-(1,3-dithietan-2-ylidene)-2-[N-(4-methylthiazol-2-yl)carbamoyl]acetate can effectively reduce the skin-tumors induced by B[a]P and VC.

While the invention has been described with respect to the specific embodiments, it should be recognized that various modifications and changes may be made by those skilled in the art to the invention which also fall within the scope of the invention as defined by the appended claims.

## Claims

1. A use of isopropyl 2-(1,3-dithietan-2-ylidene)-2-[N-(4-methylthiazol-2-yl)carbamoyl]acetate and its pharmaceutically acceptable salts in the manufacture of medicine for the prevention of cancer.
